# EUROPEAN PATENT APPLICATION

(11) **EP 4 667 574 A1**
(43) Date of publication of application: **24.12.2025**
(21) Application number: 24756177.2
(22) Date of filing: 06.02.2024
(51) Int. Cl.: C12N 15/70, C12N 15/37, C12N 15/57, C07K 14/025, C12N 9/50, A61K 39/12, A61P 31/20

(54) **TAC PROMOTER-BASED PLASMID EXPRESSION VECTOR CONSTRUCTION AND USE THEREOF**

(30) Priority: 15.02.2023 CN 202310114443
(71) Applicant: Beijing Health Guard Biotechnology, Inc., Beijing 100176 (CN)
(72) Inventor: WU, Shuming, Beijing 100176 (CN); CHEN, Xiao, Beijing 100176 (CN); LIU, Yongjiang, Beijing 100176 (CN); ZHANG, Haijiang, Beijing 100176 (CN); XUE, Junlian, Beijing 100176 (CN); WANG, Xuehong, Beijing 100176 (CN); GAO, Wenshuang, Beijing 100176 (CN); ZHANG, Ruixia, Beijing 100176 (CN); JIANG, Xulin, Beijing 100176 (CN); SHEN, Ercui, Beijing 100176 (CN); LIU, Yuying, Beijing 100176 (CN); YU, Hongyang, Beijing 100176 (CN)
(74) Representative: Karakatsanis, Georgios
(86) International application number: PCT/CN2024/076492
(87) International publication number: WO 2024/169804

(57) **Abstract**

The present invention relates to a transformation and construction method of a plasmid expression vector based on a Tac promoter, in which a Shine-Dalgarno (SD) sequence of the plasmid expression vector is replaced and a tag sequence is further knocked out, a use of such constructed expression vector in the expression of recombinant exogenous proteins, especially in the expression of human papillomavirus L1 antigen protein, and a use of the human papillomavirus L1 antigen protein expression product in the prevention of cervical cancer.

## Description

### TECHNICAL FIELD

The present invention relates to a transformation and construction method of a plasmid expression vector based on a Tac promoter, a use of the expression vector constructed by the method in the expression of recombinant proteins, especially in the expression of human papillomavirus L1 antigen protein, and a use of the human papillomavirus L1 antigen protein expression product in the prevention of cervical cancer.

### BACKGROUND

A plasmid vector is a circular DNA with a smaller relative molecular mass (generally around 1-200 kb in size, with kb representing kilobase pairs), which is independent of chromosomal DNA. There is one plasmid vector in each of some bacteria, while there are a plurality of plasmid vectors in each of other bacteria. Plasmids can be transferred from one bacterium to another through conjugation between bacteria, and can be replicated independently, or integrated into bacterial chromosomal DNA and replicated with the replication of the chromosomal DNA.

The most common expression vector for *Escherichia coli* is a plasmid vector. As an expression vector, it must first meet the basic requirements of a cloning vector, which are the ability to deliver exogenous genes into *Escherichia coli* cells. The expression vector is formed by adding expression elements on the basis of the basic skeleton of the cloning vector. The differences between various expression vectors are often manifested in the differences of their expression elements. Plasmid expression vectors of *Escherichia coli* mainly include two types: expression vectors for expressing fusion proteins and expression vectors for expressing non-fusion proteins.

A fusion protein expression vector means that a target protein is expressed in the form of a fusion protein, and the target protein can be isolated and purified by using the special properties of protein or polypeptide encoded by the vector. The part of the fusion protein co-expressed with the target protein is also called tag protein or tag polypeptide (Tag), and glutathione S-transferase (GST), hexahistidine peptide (polyHis-6), protein A and maltose binding protein (MBP) are commonly used.

A key issue in the vector construction process of fusion protein expression is the linker sequence (Linker) between two proteins, namely the linker peptide, whose length is crucial for protein folding and stability. If the linker sequence is too short, it may affect the folding of the advanced structures of the two proteins, so that the structures will interfere with each other to lead a failure in formation of a correct spatial structure. If the linker sequence is too long, it will also involve the issue of immunogenicity, because the linker sequence itself may be a new antigen. In addition, if protein drugs or vaccine products are expressed using fusion proteins, the introduction of new exogenous proteins or polypeptide (residual of tag proteins or cleavage enzymes) may increase drug safety risks.

A vector pGEX expressing fusion protein was constructed by Smith and Johnson in 1987, which is characterized by being ligated with a 26kDa GST gene; compared with other fusion vectors, it has the characteristics that the purification conditions are mild, the steps are simple without addition of a denaturing agent, and the purified protein can maintain its spatial conformation and immunogenicity to the greatest extent; and it has good application value, but the GST fusion protein tag encoded by the vector pGEX may increase the potential safety hazards of pharmaceutical protein products.

Important regulatory elements of a prokaryotic expression vector include a promoter, a Shine-Dalgarno (SD) sequence and a transcription terminator. The promoter is a segment of sequence on a DNA chain that can bind to RNA polymerase and initiate RNA synthesis. It is an indispensable and important regulatory sequence for gene expression. Without a promoter, genes cannot be transcribed. Due to the inability of bacterial RNA polymerase to recognize the promoters of eukaryotic genes, the promoters used in prokaryotic expression vectors must be prokaryotic promoters. Regulable promoters commonly used in prokaryotic expression systems include Lac (lactose promoter), Trp (tryptophan promoter), Tac (hybrid promoter of lactose and tryptophan), lPL (leftward promoter of 1-phage), T7 phage promoter, etc.

The promoter of the vector pGEX expressing the fusion protein is a Tac promoter, which is a set of hybrid promoter artificially constructed using Lac and Trp promoters. It is negatively regulated by Lac repressor protein and has a stronger initiating ability (higher expression level) than both Lac and Trp promoters. Tac1 is composed of the -35 region of the Trp promoter, a synthesized 46 bp DNA fragment (including a Pribnow box), and the operator gene in a Lac operon. Tac2 is composed of the -35 region of the Trp promoter, the -10 region of the Lac promoter, and the operator gene part in Lac operon fused with the SD sequence. The Tac promoter is induced by isopropyl-β-d-thiogalactoside (IPTG). Compared to a T7 promoter, the Tac promoter is relatively moderate in transcription intensity, slower in protein expression rate, and slower in target protein folding speed, so that a target protein expressed by the Tac promoter may be more soluble.

The SD sequence (Shine-Dalgarno sequence) was first discovered by Shine and Dalgarno in 1974. There are ribosome binding sites on mRNA, which constitute a sequence of about 3-9 bp located 3-10 bp upstream of initiation codon (AUG). This sequence is rich in purine nucleotides, which is just complementary to a pyrimidine-rich sequence at the 3' end of 16s rRNA, serving as recognition and binding sites for ribosomal RNA. Afterwards, people named this sequence Shine- Dalgarno sequence, abbreviated as SD sequence. Different SD sequences and their distances from the initiation codon (AUG) are one of the important factors affecting mRNA transcription and translation into protein. Binding of certain proteins to SD sequences will also affect the binding of mRNA to ribosomes, thereby affecting protein translation, that is, affecting the expression level of recombinant exogenous genes.

The pGEX vector is a commercial vector from General Electric (GE) in the United States, with a Tac promoter as its promoter and a SD sequence being AGGAAACAGTA.

HPV-L1 protein, which is commonly used as an antigen for human papillomavirus (HPV) vaccines, is difficult to be efficiently expressed as HPV-L1 soluble protein by *Escherichia coli,* and it is prone to form inclusion bodies during recombinant expression. Alternatively, HPV VLP can also be obtained after the above-mentioned protein is processed by the steps such as inclusion body purification and renaturation(Kelsall, S.R.and J.K.Kulski. Expression of the major capsid protein of human papillomavirus type 16 in Escherichia coli.1995.JVirol Methods 53(1):75-90), but there is a large amount of protein loss and the yield is low, making it difficult for application in large-scale production. Although HPV L1 protein can also be expressed soluble in the correct conformation in *Escherichia coli* and dissolved in the lysate supernatant of bacteria, the expression level is low, and there are many types and large amounts of foreign proteins in the supernatant, so it is quite difficult to purify target protein from the supernatant. GST fusion expression can increase the expression level of L1 protein in the supernatant and facilitate the purification of the target protein. As described by the inventor in the patent application publication document (CN201410683185), the fusion protein pGEX expression vector can efficiently express human papillomavirus L1 protein, and an L1 pentamer can be obtained after excision of the tag protein and purification, which can be directly applied to the preparation of a human papillomavirus pentamer vaccine. The pentamer can also be highly purified, assembled in vitro to form virus-like particles (VLPs), and then prepared into a human papillomavirus VLP vaccine.

However, the cleavage of fusion proteins often requires expensive enzymes, and at the same time, the introduction of new exogenous proteins or polypeptide (residual of tag proteins or proteases) may increase safety risks. Therefore, there is still a need in the art for an HPV VLP vaccine production technology with lower cost, higher yield and better safety.

### SUMMARY

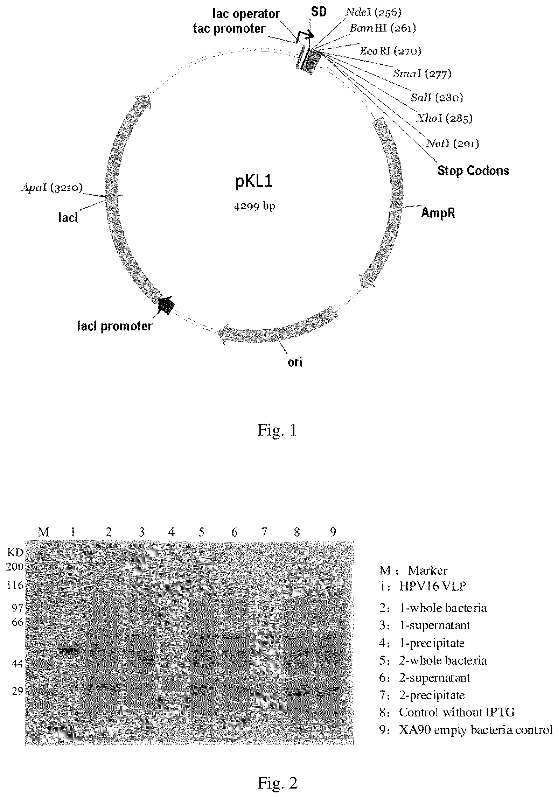

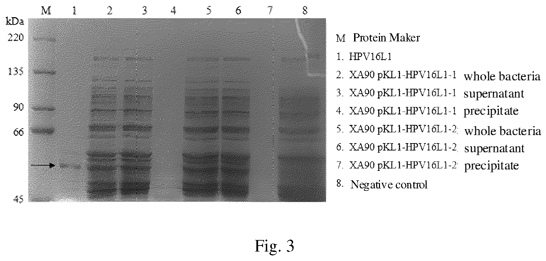

The inventor discovered unexpectedly through research that the SD sequence inherent in a pGEX plasmid vector cannot effectively achieve soluble expression of exogenous genes such as HPV16L1 or HPV18L1 in a non-fusion manner, and even if the exogenous genes are expressed, their expression levels are also very low (Fig. 2). On the basis of the pGEX plasmid vector, through the transformation of the SD sequence, a new cluster of non-fusion SD sequence expression vectors were constructed, which can efficiently express soluble exogenous proteins after transformation into *Escherichia coli,* and have the expression level and yield of L1 protein 5 to 10 times that of a fusion protein expression technology (Fig. 3) especially in terms of human papillomavirus L1 antigen protein expression, thus making it possible to realize large-scale industrial production of cervical cancer vaccines. The present invention has now been accomplished based on the above-mentioned invention.

Therefore, the first aspect of the present invention relates to the transformation of a cluster of expression vectors, which is the transformation of a plasmid expression vector based on a Tac promoter. More specifically, the promoter of pGEX series products is Tac, and the pGEX series products include pGEX-1λT, pGEX-2T, pGEX-2TK, pGEX-3X, pGEX-4T-1, pGEX-4T-2, pGEX-4T-3, pGEX-5X-1, pGEX-5X-2, pGEX-5X-3, pGEX-6P-1, pGEX-6P-2, pGEX-6P-3, and recombinant vectors transformed from the above pGEX series products. The expression vectors are obtained by deleting the coding sequences of GST fusion proteins in pGEX vectors and transforming the SD sequences. The pKL1 expression vector obtained by transformation is shown in Fig. 1.

The pGEX vectors involved in the present invention include pGEX series products, such as pGEX-1λT, pGEX-2T, pGEX-2TK, pGEX-3X, pGEX-4T-1, pGEX-4T-2, pGEX-4T-3, pGEX-5X-1, pGEX-5X-2, pGEX-5X-3, pGEX-6P-1, pGEX-6P-2, pGEX-6P-3, and recombinant vectors transformed from the above pGEX series products.

The original SD sequence of the pGEX vector in the present invention is: AGGAAACAGTA.

In one embodiment, the SD sequence of the pGEX-6P-2 vector is converted into AGGAGATATA, the GST gene is knocked out at the same time, and the resulting vector is called pKL1; the SD sequence of the pGEX-4T-1 vector is converted into AGGAAACAGTA, the GST gene is knocked out at the same time, and the resulting vector is called pKL10; the SD sequence of the pGEX-2TK vector is converted into AGGAGGAATAA, the GST gene is knocked out at the same time, and the resulting vector is called pKL20; and the SD sequence of the pGEX-5X-2 vector is converted into AGGAGGAATTA, the GST gene is knocked out at the same time, and the resulting vector is called pKL30.

In the study, the inventor also made other attempts to transform the vectors. The replaced SD sequences are listed as follows: AGGAAACAGCT, named pEZSeq-Kan; AGGAAGCTAAA, named pDNR-Dual; AGGAAGACT, named pBR322; AGGAGATATACAT, named pRSET A; AGGGGTGTT, named pSTBlue; and AGGAGGTTGT, named pEX3. However, their effects were not satisfactory and were abandoned.

The second aspect of the present invention relates to providing a cluster of recombinant plasmids, including the above-mentioned expression vectors and exogenous genes that are inserted into the expression vectors and encode exogenous proteins. For example, the exogenous gene includes, but not limited to the gene encoding an antigenic protein or polypeptide of microorganisms such as viruses or bacterium, and the gene encoding a tumor antigen, wherein the exogenous gene is selected from the group consisting of a DNA sequence encoding human papillomavirus L1 protein (HPVL1), a gene encoding human rhinovirus 3C protease (HRV 3C, abbreviated as 3C protease), a gene encoding human enterovirus 71 (EV71) capsid protein, a gene encoding norovirus (NoV) capsid protein, and the like.

The third aspect of the present invention relates to providing an expression method, including a host cell, and the expression vectors of the present invention or the recombinant plasmids of the present invention as described above. The host cell is *Escherichia coli* including, but not limited to DH5α, GI698, ER2566, BL21(DE3), XA90, B834(DE3) or BLR(DE3).

The fourth aspect of the present invention relates to providing a method for constructing an expression vector by genetic recombination, comprising the steps of:
S1. designing mutagenic primers according to an SD sequence;
S2. performing mutagenic polymerase chain reaction (PCR) using a pGEX vector as a template to introduce an NdeI restriction site into the original vector;
S3. removing GST gene sequence by NdeI and BamHI double digestion;
S4. filling in cohesive ends with DNA polymerase I and Klenow fragments (i.e., Large (Klenow) Fragment), where the Klenow fragment is a fragment at the C-terminus of DNA polymerase I;
S5. ligating with T4 DNA ligase to obtain a closed-circular plasmid;
S6. designing primers for PCR to reintroduce the NdeI restriction site into the plasmid; and
S7. expanding a clone with a correct sequence, preserving the bacterial strain, and extracting and storing the plasmid to obtain the vector of the present invention.

The fifth aspect of the present invention relates to use of the new SD expression vector of the present invention to the efficient expression of various pharmaceutical recombinant proteins, including L1 protein and its virus-like particles (VLPs) applicable for the preparation of human papillomavirus vaccine antigens HPV6, HPV11, HPV16, HPV18, HPV31, HPV33, HPV35, HPV39, HPV45, HPV51, HPV52, HPV56, HPV58, HPV59 and HPV68, which can be applied to the preparation of human rhinovirus 3C protease, human enterovirus 71 (EV71) capsid protein and VLP, norovirus (NoV) capsid protein and VLP, poliovirus capsid protein and VLP, hepatitis A virus (HAV) capsid protein and VLP, hepatitis B virus (HBV) capsid protein and VLP, and the like. Before the implementation of the present invention, none of these exogenous proteins could be expressed soluble in *Escherichia coli* cells, or these exogenous proteins were very low in soluble expression levels.

Beneficial effects of the present invention: In prokaryotic expression systems, an *Escherichia coli* expression system has the advantages of low culture cost and high expression level. However, the HPV L1 protein expressed in the *Escherichia coli* expression system often loses its correct natural conformation and is expressed in the form of inclusion bodies in the precipitate. At present, renaturation of proteins expressed in inclusion bodies still remains a global challenge. The renaturation is difficult and inefficient, which makes it difficult to implement VLP with correct conformation obtained from inclusion bodies in large-scale production and causes the VLP to be only limited to small-scale laboratory research. Although HPV L1 can also be expressed with correct conformation in a soluble form in *Escherichia coli* lysate supernatant, its expression level is low, and it is quite difficult to purify HPV L1 protein from a wide variety of soluble proteins in the *Escherichia coli* lysate supernatant. The purification often needs to be carried out by means of fusion expression and affinity chromatography, which often requires expensive enzymes and thus causes drug safety problems.

The novel SD plasmid expression vector constructed in the present invention is highly capable of efficiently expressing HPV L1 protein in the *Escherichia coli* expression system. The purification method used does not require expensive enzymes and does not involve tag protein, thereby eliminating potential drug safety hazards caused by the tag protein. The high-purity HPV L1 protein obtained through further purification can be assembled into virus-like particles with good immunogenicity, which can induce high-titer neutralizing antibodies against HPV and prevent HPV infection in the human body. It is a good form of vaccine.

In addition, the present invention unexpectedly discovered that the novel SD sequence expression vector constructed using the above-mentioned technology, after transformation into *Escherichia coli,* has the expression level and yield of L1 protein 5 to 10 times that of a fusion protein expression technology in terms of human papillomavirus L1 antigen protein expression, thus making it possible to realize low-cost and large-scale industrial production of cervical cancer vaccines. For example, the expression vector is used for preparing a human papillomavirus vaccine L1 for HPV6, HPV11, HPV16, HPV18, HPV31, HPV33, HPV35, HPV39, HPV45, HPV51, HPV52, HPV56, HPV58, HPV59 and HPV68, and VLP antigen proteins.

On the other hand, the novel SD plasmid expression vector constructed by the present invention can achieve efficient soluble expression of various exogenous proteins by cooperating with appropriate host cells. For example, it can be applied to the expression of human rhinovirus 3C protease, the expression of human enterovirus 71 (EV71) capsid protein and VLP, the expression of norovirus (NoV) capsid protein and VLP, the expression of poliovirus capsid protein and VLP, and the expression of hepatitis A virus (HAV) capsid protein and VLP as well as hepatitis B virus (HBV) capsid protein and VLP.

These inventive features and novel aspects of the present invention will become apparent upon reference to the following detailed description and accompanying drawings.

### BRIEF DESCRIPTION OF THE DRAWINGS

Fig. 1 shows a schematic diagram of an expression vector pKL1. The schematic diagrams of expression vectors pKL10, pKL20, and pKL30 are similar to that of pKL1.
Fig. 2 shows electrophoresis detection results of HPV16 L1 protein expression before SD sequence replacement and transformation, without L1 protein expression. The results of two experiments are shown.
Fig. 3 shows electrophoresis detection results of HPV16 L1 protein expression after SD sequence replacement and transformation, with L1 protein being highly expressed. The results of two experiments are shown.
Fig. 4 shows detection results of highly soluble expression of HRV 3C protease in Embodiment 6.

### DETAILED DESCRIPTION OF THE EMBODIMENTS

The present invention provides an expression vector, which contains all expression elements capable of achieving non-fusion protein expression of a variety of exogenous genes.

### Embodiment 1 Construction of pKL series vectors

### 1.1 Introduction of NdeI restriction site into pGEX-6P-2 plasmid by mutagenic PCR:

The names and sequences of PCR primers were as follows:
Forward primer: 6p1-NdeImut-F (5' to 3') 
ATTTCA CACAGG AAACAG TACATA TGTCCC CTATAC TAGGTT
ATTGGA AAATTA AG;
Reverse primer: 6p1-NdeImut-R sequence (5' to 3')

ATAACC TAGTAT AGGGGA CATATG TACTGT TTCCTG TGTGAA
ATTGTT ATCC.

A PCR reaction system was as follows: 10 µL of 5×phusion HF buffer, 30.5 µL of ddH₂O, 2 µL of 10 mM dNTP, 1 µL of 6PNE-SDm-F, 1 µL of 6PNE-SDm-R, 5 µL of pGEX-6P-2 (diluted 20 times), and 0.5 µL of Phusion HF Enzyme.

PCR reaction program settings: 95°C for 3 min; 95°C for 1 min, 55°C for 1 min, and 72°C for 10 min; 20 cycles; and 72°C for 15 min.

A PCR product was digested with DpnI and then transformed into *Escherichia coli* DH5α. Monoclonal colonies were obtained after overnight culture. The monoclonal colonies were expanded and cultured, and then their vector sequences were sequenced by a professional gene sequencing company to select clones with correct sequencing results. After that, the clones were propagated and plasmids were extracted therefrom to obtain a vector into which an NdeI restriction site was successfully introduced.

1.2 A mutant PCR primer was designed to replace an SD sequence, and then the SD sequence of the original vector was replaced by a PCR method.

The primer information was as follows:
6PNE-SDm-F (5' to 3'):
   CAATTTCACACAGGAGATATACATATGTCCCCTATACTAGG
6PNE-SDm-R (5' to 3'):
   GTATAGGGGACATATGTATATCTCCTGTGTGAAATTGTTATCC

A PCR reaction system was as follows: 10 µL of 5×phusion HF buffer, 30.5 µL of ddH₂O, 2 µL of 10 mM dNTP, 1 µL of 6PNE-SDm-F, 1 µL of 6PNE-SDm-R, 5 µL of the plasmid obtained in step 1.1, and 0.5 µL of Phusion HF Enzyme.

PCR reaction program settings: 95°C for 3 min; 95°C for 1 min, 55°C for 1 min, and 72°C for 10 min; 20 cycles; and 72°C for 15 min.

After the template DNA of a PCR product was digested with DpnI, the PCR product was transformed into *Escherichia coli* DH5α. Monoclonal colonies were obtained after overnight culture. The monoclonal colonies were expanded and cultured, and then their vector sequences were sequenced by a professional gene sequencing company to select clones with correct sequencing results. After that, the clones were propagated and plasmids were extracted therefrom to obtain a vector with a successfully replaced SD sequence.

### 1.3 Removal of GST gene by NdeI and BamHI double digestion of vector

A digestion system was as follows: 3 µl of Cutsmart buffer, 3 µl of ddH₂O, 20 µl of the vector obtained in step 1.2, 2 µl of NdeI, and 2 µl of BamHI.

Enzymatic digestion was carried out at 37°C for 2 h; 0.8% agarose gel electrophoresis was performed under 120 V for 1 h; and the gel was cut to obtain an electrophoresis band corresponding to the vector fragment with a GST gene being removed, which was stored at 4°C.

The vector fragment was recovered by an agarose gel recovery kit, and 3 µl of the obtained vector fragment was taken for electrophoresis to detect the recovery result. Then, cohesive ends of a double digestion product were filled in with DNA polymerase I, and a reaction system was as follows: 2.5 µl of 10X T4 DNA ligase buffer, 1.8 µl of ddH₂O, 20 µl of the enzyme-digested vector fragment obtained after glue recovery, 0.2 µl of 10 mM dNTP, and 0.5 µl of DNA polymerase I; and a reaction was carried out at 25°C for 15 min, and ethylene diamine tetraacetic acid (EDTA) (with a final concentration of 10 mM) was added and heated at 75°C for 20 min to terminate the reaction.

The enzyme-digested and end-filled vector was re-ligated and circularized, and a ligation system was as follows: 2 µl of T4 DNA Ligase buffer, 16 µl of a linear blunt-ended vector fragment, and 2 µl of T4 DNA ligase. Ligation was carried out at 16°C for 4 h.

A ligation product was digested and then transformed into *Escherichia coli* DH5α. Monoclonal colonies were obtained after overnight culture. The monoclonal colonies were expanded and cultured, and then their vector sequences were sequenced by a professional gene sequencing company to select clones with correct sequencing results. After that, the clones were propagated and plasmids were extracted therefrom to obtain a vector, with an SD sequence being successfully replaced and a GST gene being removed.

The plasmid was amplified by PCR, and the restriction sites of NdeI and BamHI were reintroduced.

PCR primers were as follows:
6PNE-SDm-noG-F (5' to 3'):
   CAGGAGATATACATATGGGATCCCCGGAATTCCCG
6PNE-SDm-noG-R (5' to 3'):
   GAATTCCGGGGATCCCATATGTATATCTCCTGTGTG

A PCR reaction system was as follows: 10 µL of 5×phusion HF buffer, 30.5 µL of ddH₂O, 2 µL of 10 mM dNTP, 1 µL of 6PNE-SDm-noG-F, 1 µL of 6PNE-SDm-R, 5 µL of template plasmid, and 0.5 µL of Phusion HF Enzyme.

PCR reaction program settings: 95°C for 3 min; 95°C for 1 min, 55°C for 1 min, and 72°C for 10 min; 20 cycles; and 72°C for 15 min.

After the template DNA of a PCR product was digested with DpnI, the PCR product was transformed into *Escherichia coli* DH5α. Monoclonal colonies were obtained after overnight culture. The monoclonal colonies were expanded and cultured, and then their vector sequences were sequenced by a professional gene sequencing company to select clones with correct sequencing results. After that, the clones were propagated and plasmids were extracted therefrom to obtain a vector, with an SD sequence being successfully replaced and a GST gene being removed, and NdeI and BamHI were reintroduced into the vector. Thus, the construction of the pKL1 vector was completed.

Referring to the above experimental steps, pGEX-2TK was used as a starting vector to replace pGEX-6P-2 in the above experimental steps, with SEQ ID No. 3 being a new SD sequence for replacement to construct pKL20. Mutagenic primers based on the SD sequence were designed as follows:
Forward primer (5' to 3')
   CAATTTCACACAGGAAGGAGGAATAACATATGCCGTCTGAAGCTAC
Reverse primer (5' to 3')
   GACGGCATATGTTATTCCTCCTTCCTGTGTGAAATTGTTATCC

Except for the starting vector and the mutagenic primers based on the SD sequence, the other steps and methods of vector construction were the same as those of pKL1 construction.

Referring to the above experimental steps, pGEX-5X-2 was used as a starting vector, with SEQ ID No. 4 being a new SD sequence for replacement to construct pKL30. Mutagenic primers based on the SD sequence were designed as follows:
Forward primer (5' to 3') CAATTTCACACAGGA AGGAGGAATTA CATATGCCGTCTGAAGCTAC
Reverse primer (5' to 3') GACGGCATATGTAATTCCTCCT TCCTGTGTGAAATTGTTATCC

Except for the starting vector and the mutagenic primers based on the SD sequence, the other steps and methods of vector construction were the same as those of pKL1 construction.

Referring to the above experimental steps, pGEX-4T-1 was used as a starting vector, with SEQ ID No. 2 being a new SD sequence for replacement to construct pKL10. Mutagenic primers based on the SD sequence were designed as follows:
Forward primer (5' to 3') CAATTTCACACAGGA AGGAAACAGTA CATATGCCGTCTGAAGCTAC
Reverse primer (5' to 3') GACGGCATATGTACTGTTTCCT TCCTGTGTGAAATTGTTATCC

Except for the starting vector and the mutagenic primers based on the SD sequence, the other steps and methods of vector construction were the same as those of pKL1 construction.

Thus, expression vectors (with tags removed) containing four different SD sequences have been successfully constructed, that is, the SD sequence in the vector pKL1 is AGGAGATATA, the SD sequence in pKL10 is AGGAAACAGTA, the SD sequence in pKL20 is AGGAGGAATAA, and the SD sequence in pKL30 is AGGAGGAATTA.

### Embodiment 2: Construction of various types of HPVL1 proteins based on vectors pKL1 and pKL30

The following various types of capsid protein L1 genes of human papillomavirus were artificially synthesized, and their sequences are shown in the sequence listing. The corresponding names of the specific sequence numbers are as follows:
The sequence as shown in SEQ ID No. 1 is HPV6L1 N/C-terminus truncated (469 aa, Theoretical pI/Mw: 5.94/52079.77 Da, N-terminus truncated by 2 amino acids, C-terminus truncated by 29 amino acids)
The sequence as shown in SEQ ID No. 2 is HPV11L1 N/C-terminus truncated (470 aa, Theoretical pI/Mw: 6.08/52274.02 Da, N-terminus truncated by 3 amino acids, C-terminus truncated by 29 amino acids)
The sequence as shown in SEQ ID No. 3 is HPV16L1 N/C-terminus truncated (472 aa, Theoretical pI/Mw: 6.08/52548.71 Da, N-terminus truncated by 4 amino acids, C-terminus truncated by 29 amino acids)
The sequence as shown in SEQ ID No. 4 is HPV18L1 N/C-terminus truncated (473 aa, Theoretical pI/Mw: 5.94/52722.42 Da, N-terminus truncated by 4 amino acids, C-terminus truncated by 30 amino acids)
The sequence as shown in SEQ ID No. 5 is HPV31L1 N/C-terminus truncated (473 aa, Theoretical pI/Mw: 6.27/52869.89 Da, N-terminus truncated by 4 amino acids, C-terminus truncated by 27 amino acids)
The sequence as shown in SEQ ID No. 6 is HPV33L1 N/C-terminus truncated (471 aa, Theoretical pI/Mw: 5.88/52765.94 Da, N-terminus truncated by 4 amino acids, C-terminus truncated by 24 amino acids)
The sequence as shown in SEQ ID No. 7 is HPV35L1 N/C-terminus truncated (470 aa, Theoretical pI/Mw: 6.10/52494.45 Da, N-terminus truncated by 4 amino acids, C-terminus truncated by 28 amino acids)
The sequence as shown in SEQ ID No. 8 is HPV39L1N9 N/C-terminus truncated (467 aa, Theoretical pI/Mw: 6.16/52393.22 Da, N-terminus truncated by 9 amino acids, C-terminus truncated by 29 amino acids)
The sequence as shown in SEQ ID No. 9 is HPV45L1 N/C-terminus truncated (476 aa, Theoretical pI/Mw: 5.94/53244.30 Da, N-terminus truncated by 4 amino acids, C-terminus truncated by 30 amino acids)
The sequence as shown in SEQ ID No. 10 is HPV51L1t N/C-terminus truncated (472 aa, Theoretical pI/Mw: 5.88/52769.56 Da, N-terminus truncated by 4 amino acids, C-terminus truncated by 28 amino acids)
The sequence as shown in SEQ ID No. 11 is HPV52L1 N/C-terminus truncated (476 aa, Theoretical pI/Mw: 5.88/53176.26 Da, N-terminus truncated by 4 amino acids, C-terminus truncated by 23 amino acids)
The sequence as shown in SEQ ID No. 12 is HPV56L1t N/C-terminus truncated (470 aa, Theoretical pI/Mw: 6.11/52796.70 Da, N-terminus truncated by 4 amino acids, C-terminus truncated by 25 amino acids)
The sequence as shown in SEQ ID No. 13 is HPV58L1 N/C-terminus truncated (471 aa, Theoretical pI/Mw: 5.80/52994.97 Da, N-terminus truncated by 4 amino acids, C-terminus truncated by 23 amino acids)
The sequence as shown in SEQ ID No. 14 is HPV59L1 N/C-terminus truncated (473 aa, Theoretical pI/Mw: 6.08/52891.89 Da, N-terminus truncated by 4 amino acids, C-terminus truncated by 31 amino acids)
The sequence as shown in SEQ ID No. 15 is HPV68L1a N/C-terminus truncated (473 aa, Theoretical pI/Mw: 5.75/53032.99 Da, N-terminus truncated by 4 amino acids, C-terminus truncated by 28 amino acids)

First, the DNA fragment of HPV L1 was amplified by PCR, and the information of primers used was as shown in Table 1.

**Table 1 Primer information**

| **L1 sequence** | **Primer information** |
|---|---|
| HPV6L1 (SEQ ID No. 1) | 5' primer: ACTTCAGGGCCCTCTGACTCTACCGTTTACGTTCCG |
| | 3' primer: ATCTCACTCGAGCTAACCACGGTAACCAGACTGCAGC |
| HPV11L1 (SEQ ID No. 2) | 5' primer: ACTTCAGGGCCCTCTGACTCTACCGTTTACGTTCC |
| | 3' primer: ATCTCACTCGAGCTAACCACGGTAACCAGACTGCAGC |
| HPV16L1 (SEQ ID No. 3) | 5' primer: ACTTCAGGGCCCTCTGAAGCTACCGTTTACCTGC |
| | 3' primer: ATCTCACTCGAGCTAAGCTTTCAGACCAGCCTGCAGC |
| HPV18L1 (SEQ ID No. 4) | 5' primer: ACTTCAGGGCCCTCTGACAACACCGTTTACCTGCC |
| | 3' primer: ATCTCACTCGAGCTAACGACGCAGACCAGCCTGAACC |
| HPV31L1 (SEQ ID No. 5) | 5' primer: ACTTCAGGGCCCTCTGAAGCTACCGTTTACCTGC |
| | 3' primer: ATCTCACTCGAGCTAAGCACGGTAACCAGCCTGCAGC |
| HPV33L1 (SEQ ID No. 6) | 5' primer: ACTTCAGGGCCCTCTGAAGCTACCGTTTACCTGC |
| | 3' primer: ATCTCACTCGAGCTAAGCTTTCAGACCAGCCTGCAGC |
| HPV35L1 (SEQ ID No. 7) | 5' primer: GGGAATTCCATATGTCTAACGAAGCTACCGTTTACCTG |
| | 3' primer: ATCTCACTCGAGCTAAGCTTTCAGACCAGCCTGCAGC |
| HPV39-N9 L1 (SEQ ID No. 8) | 5' primer: GGGAATTCCATATGGTTTACCTGCCGCCGCCGTC |
| | 3' primer: ATCTCACTCGAGCTAACGACGAACACGAGCCTGCAG |
| HPV45L1 (SEQ ID No. 9) | 5' primer: ACTTCAGGGCCCTCTGACTCGACCGTATACCTGC |
| | 3' primer: ATCTCACTCGAGCTAACGACGCAGACCAGCCTGAACC |
| HPV51L1t (SEQ ID No. 10) | 5' primer:GGAATTCCATATGGCTCTGT GGCGTACCAA CG |
| | 3' primer: ATCTCACTCGAGCTAACGCTGAACACCAACCTGCAG |
| HPV52L1 (SEQ ID No. 11) | 5' primer: ACTTCAGGGCCCTCTGAAGCTACCGTTTACCTGC |
| | 3' primer: ATCTCACTCGAGCTAAGCCTGCAGACCAGCCTGCAGC |
| HPV56Llt (SEQ ID No. 12) | 5' primer: GGAATTCCATATGGCTACCT GGCGTCCGTC TG |
| | 3' primer: ATCTCACTCGAGCTAAGAACGGGTACCCAGCTGCATC |
| HPV58L1 (SEQ ID No. 13) | 5' primer: ACTTCAGGGCCCTCTGAAGCTACCGTTTACCTGC |
| | 3' primer: ATCTCACTCGAGCTAAGCTTTCAGACCAGACTGCAGC |
| HPV59L1 (SEQ ID No. 14) | 5' primer: GGAATTCCATATGGCTCTGT GGCGTTCTTC TG |
| | 3' primer: ATCTCACTCGAGCTACGGACGAGCACCCAGCTGC |
| HPV68L1a (SEQ ID No. 15) | 5' primer: GGAATTCCATATGGCTCTGT GGCGTTCTTC TG |
| | 3' primer: ATCTCACTCGAGCTAACGACGAACACCAGCCTGCAG |

The L1 gene PCR fragment containing NdeI and Xho1 restriction sites and the recombinant vector pKL1 or pKL30 were separately subjected to NdeI/Xho1 double digestion, and then the recovered gene fragment was ligated with pKL1 or pKL30 containing the corresponding cohesive ends using T4 DNA ligase at 16°C for 10-15 h. The ligation system was as follows: 6 µl of pKL1 or pKL30 vector fragment, 2 µl of L1 gene fragment, 1 µl of T4 DNA ligase, and 1 µl of T4 DNA Ligase buffer.

After the ligation reaction, a ligation product was transformed into *Escherichia coli* DH5α for screening of recombinants. The screened monoclonal colonies were expanded and cultured and plasmids were extracted, followed by sequencing validation to obtain recombinant expression vectors pKL1-HPVL1 and pKL30-HPVL1.

The recombinant vectors with correct sequencing results were transformed into *Escherichia coli* XA90 host cells, and used as engineering bacteria expressing recombinant proteins for expression of HPV L1 protein. An engineering bacteria activation medium was a Luria-Bertani (LB) medium (10 g/L tryptone; 5 g/L yeast powder; 10 g/L NaCl), and an expression medium was a 2YT medium (16 g/L tryptone; 10 g/L yeast powder; 5 g/L NaCl).

Engineering bacteria strains were inoculated into the LB medium (Amp+) at an inoculation amount of 0.05%, and cultured at 37°C and 220 rpm for 16 h for activation. The activated bacterial solution was inoculated into the 2YT medium at an inoculation amount of 0.5%, cultured at 30°C and 220 rpm for 7 h, then added with IPTG with a final concentration of 0.2 mM, and induced and cultured at 30°C and 220 rpm for 16 h to terminate fermentation; and bacterial cells were collected by centrifugation for expression level detection and purification experiments.

The result of SDS-PAGE takes the expression of HPV16 L1 protein as an example. The electrophoresis detection results of HPV16 L1 protein expression before SD sequence replacement and transformation (without L1 protein expression) are shown in Fig. 2, while Fig. 3 shows the electrophoresis detection results of HPV16 L1 protein expression after SD sequence replacement and transformation (with L1 protein being highly expressed). The quantified protein expression is shown in the following table:

**Table 2 Summary of L1 protein expression levels before and after vector transformation**

| **L1 sequence** | **Transformed expression vector** | **Expression vector before transformation** | **Protein expression level before transformation (mg/g wet bacterial cells)** | **Protein expression level after transformation (mg/g wet bacterial cells)** |
|---|---|---|---|---|
| HPV6L1 | pKL1 | pGEX-6P-2 | 0 | 0.91 |
| HPV11L1 | pKL1 | pGEX-6P-2 | 0 | 1.73 |
| HPV16L1 | pKL1 | pGEX-6P-2 | 0 | 6.77 |
| HPV18L1 | pKL1 | pGEX-6P-2 | 0 | 0.7 |
| HPV31L1 | pKL1 | pGEX-6P-2 | 0 | 4.14 |
| HPV33L1 | pKL1 | pGEX-6P-2 | 0 | 0.68 |
| HPV35L1 | pKL1 | pGEX-6P-2 | 0 | 1.55 |
| HPV39-N9 L1 | pKL1 | pGEX-6P-2 | 0 | 0.59 |
| HPV45L1 | pKL1 | pGEX-6P-2 | 0 | 1.04 |
| HPV51L1t | pKL1 | pGEX-6P-2 | 0 | 0.35 |
| HPV52L1 | pKL1 | pGEX-6P-2 | 0 | 0.20 |
| HPV56L1t | pKL1 | pGEX-6P-2 | 0 | 0.43 |
| HPV58L1 | pKL1 | pGEX-6P-2 | 0 | 0.96 |
| HPV59L1 | pKL30 | pGEX-5X-2 | 0 | 2.41 |
| HPV68L1a | pKL30 | pGEX-5X-2 | 0 | 2.07 |

From the results, it can be seen that the vector is basically not expressed before transformation, the protein expression level of the transformed vector reaches at least 0.2 mg/g wet bacterial cells, and the highest expression level is 6.77 mg/g wet bacterial cells.

### Embodiment 3 Expression of HPV16L1 antigen protein by different pKL series vectors-HPV16 L1 expression vector

The recombinant vector HPV16L1 with correct sequencing results in Embodiment 2 was transformed into *Escherichia coli* XA90 host cells, and used as engineering bacteria expressing recombinant proteins for expression of HPV L1 protein. Engineering bacteria strains were inoculated into the LB medium (Amp+) at an inoculation amount of 0.05%, and cultured at 37°C and 220 rpm for 16 h for activation. The activated bacterial solution was inoculated into the 2YT medium at an inoculation amount of 0.5%, cultured at 30°C and 220 rpm for 7 h, then added with IPTG with a final concentration of 0.2 mM, and induced and cultured at 30°C and 220 rpm for 16 h to terminate fermentation; and bacterial cells were collected by centrifugation for expression level detection and purification experiments.

The results are shown in Table 3, where it can be seen that two of the SD sequences are expressed in a large amount, while the other two SD sequences are almost not expressed.

**Table 3 Comparison table of target protein expression levels in HPV16 L1 engineering bacteria containing different SD sequences**

| **Engineering bacteria** | **SD sequence** | **Target protein expression level (mg/g wet bacterial cells)** |
|---|---|---|
| XA90 pKL1-HPV16L1 | AGGAGATATA | 6.77 |
| XA90 pKL10-HPV16L1 | AGGAAACAGTA | 6.77 |
| XA90 pKL20-HPV16L1 | AGGAGGAATAA | 0 |
| XA90 pKL30-HPV16L1 | AGGAGGAATTA | 0 |

### Embodiment 4 Expression of HPV59 L1 antigen protein by different pKL series vectors-HPV59 L1 expression vector

The recombinant vector HPV59L1 with correct sequencing results in Embodiment 2 was transformed into *Escherichia coli* XA90 host cells, and used as engineering bacteria expressing recombinant proteins for expression of HPV L1 protein. Engineering bacteria strains were inoculated into the LB medium (Amp+) at an inoculation amount of 0.05%, and cultured at 37°C and 220 rpm for 16 h for activation. The activated bacterial solution was inoculated into the 2YT medium at an inoculation amount of 0.5%, cultured at 30°C and 220 rpm for 7 h, then added with IPTG with a final concentration of 0.2 mM, and induced and cultured at 30°C and 220 rpm for 16 h to terminate fermentation; and bacterial cells were collected by centrifugation for expression level detection and purification experiments.

The results are shown in Table 4, where it can be seen that there is a relatively large amount of expression when one of the SD sequences is used, while there is almost no expression when the other three SD sequences are used.

**Table 4 Comparison table of target protein expression levels in HPV59 L1 engineering bacteria containing different SD sequences**

| **Engineering bacteria** | **SD sequence** | **Target protein expression level (mg/g wet bacterial cells)** |
|---|---|---|
| XA90 pKL1-HPV59L1 | AGGAGATATA | 0 |
| XA90 pKL10-HPV59L1 | AGGAAACAGTA | 0 |
| XA90 pKL20-HPV59L1 | AGGAGGAATAA | 0 |
| XA90 pKL30-HPV59L1 | AGGAGGAATTA | 2.41 |

### Embodiment 5 Expression of HPV68 L1 antigen protein by different pKL series vectors-HPV68 L1 expression vector

The recombinant vector HPV68 L1 with correct sequencing results in Embodiment 2 was transformed into *Escherichia coli* XA90 host cells, and used as engineering bacteria expressing recombinant proteins for expression of HPV L1 protein. Engineering bacteria strains were inoculated into the LB medium (Amp+) at an inoculation amount of 0.05%, and cultured at 37°C and 220 rpm for 16 h for activation. The activated bacterial solution was inoculated into the 2YT medium at an inoculation amount of 0.5%, cultured at 30°C and 220 rpm for 7 h, then added with IPTG with a final concentration of 0.2 mM, and induced and cultured at 30°C and 220 rpm for 16 h to terminate fermentation; and bacterial cells were collected by centrifugation for expression level detection and purification experiments.

The results are shown in Table 5, where it can be seen that there is a relatively large amount of expression when one of the SD sequences is used, while there is almost no expression when the other three SD sequences are used.

**Table 5 Comparison table of target protein expression levels in HPV68 L1 engineering bacteria containing different SD sequences**

| **Engineering bacteria** | **SD sequence** | **Target protein expression level (mg/g wet bacterial cells)** |
|---|---|---|
| XA90 pKL1-HPV68Lla | AGGAGATATA | 0 |
| XA90 pKL10-HPV68L1a | AGGAAACAGTA | 0 |
| XA90 pKL20-HPV68L1a | AGGAGGAATAA | 0 |
| XA90 pKL30-HPV68L1a | AGGAGGAATTA | 2.07 |

### Embodiment 6 Construction of human rhinovirus 3C protease expression vector pKL1-HRV3C

The human rhinovirus 3C protease (HRV 3C) gene is artificially synthesized, and its specific sequence is as shown in SEQ ID No. 16. Firstly, the DNA fragment of HRV 3C was amplified by PCR, and the primer information was as follows: (the restriction sites were NdeI and XhoI):
Forward primer P3C-NdeI:
   GGAATTCCATATGGGACCAAACACAGAATTTGCAC
Reverse primer P3C-XhoI:
   GGTCTCGAGTTATTGTTTCTCTACAAAATATTG

The L1 gene PCR fragment containing NdeI and Xho1 restriction sites and the transformed expression vector pKL1 were separately subjected to NdeI/Xho1 double digestion, and then the recovered gene fragment was ligated with pKL1 containing the corresponding cohesive ends using T4 DNA ligase at 16°C for 10-15 h.

The ligation system was as follows: 6 µl of pKL1 vector fragment, 2 µl of 16 L1 gene fragment, 1 µl of T4 DNA ligase, and 1 µl of T4 DNA Ligase buffer.

Re-transformation identification: After the ligation reaction, a ligation product was transformed into *Escherichia coli* DH5α for screening of recombinants. The screened monoclonal colonies were expanded and cultured and plasmids were extracted, followed by sequencing validation to obtain a recombinant expression vector pKL1-HRV3C.

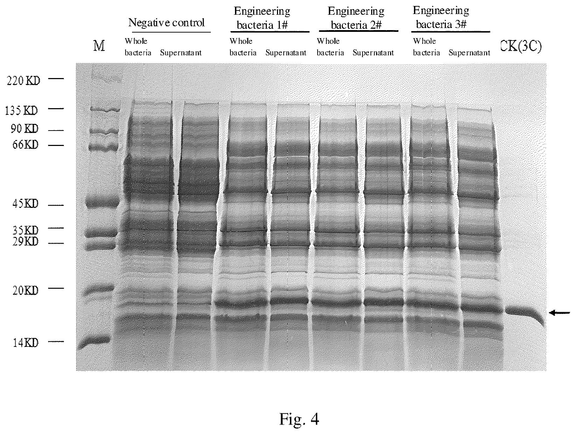

The expression and results of recombinant HRV 3C protease were detected according to following steps:
(1) The expression vector pKL1-3C was transformed into *Escherichia coli* host bacteria, and the empty vector pKL1 was used to transform the host bacteria at the same time, serving as a negative control bacteria.
(2) Monoclonal streaking and inoculation for expression of engineering bacteria: Three clones were selected from a plasmid transformation plate obtained by transforming the host bacteria with pKL1-3C, the clones were respectively picked up with toothpicks for streaking and passage, and then the streaked toothpicks were put into a 40 ml LB liquid medium containing 100 µg/ml Amp. For a control group, a single colony was selected from the transformation plate obtained by transforming the host bacteria with the empty vector pKL1. After streaking, it was also inoculated into a 40 ml LB medium containing 100 µg/ml Amp. The culture conditions were 37°C, 220 rpm, and shaking overnight.
(3) Lactose induction: The overnight cultured bacterial solution was transferred to a 2YT fermentation medium at an inoculation amount of 0.5%, cultured at 37°C and 220 rpm until the OD600 of the bacterial solution was 1.5-2.0, then added with lactose to a final concentration of 2 g/L, and subjected to overnight induction at 30°C and 220 rpm.
(4) The cells were disrupted by ultrasonic waves, and then the expression results were detected by SDS-PAGE electrophoresis to analyze whether the target protein was expressed and whether it was soluble expression. The results are shown in Fig. 4 (parallel experiments of three single colony engineering bacteria 1# to 3#), which demonstrate that the method provided by the present invention can enable the massive soluble expression of HRV 3C protease.

Finally, it should be noted that the foregoing descriptions are merely preferred embodiments of the present invention, and are not intended to limit the present invention. Although the present invention has been described in detail with reference to the aforementioned embodiments, those skilled in the art may still modify the technical solutions described in the aforementioned various embodiments, or equivalently replace some of the technical features therein. Any modification, equivalent replacement, improvement and the like made within the spirit and principle of the present invention shall be included within the protection scope of the present invention.

## Claims

1. A plasmid expression vector based on a Tac promoter, **characterized in that** a Shine-Dalgarno (SD) sequence of the plasmid expression vector is AGGAGATATA, AGGAAACAGTA, AGGAGGAATAA, and AGGAGGAATTA.

2. The plasmid expression vector according to claim 1, **characterized in that** a framework of the plasmid expression vector based on the Tac promoter is a pGEX vector, wherein a glutathione S-transferase (GST) gene is knocked out.

3. A use of the plasmid expression vector according to claim 1 or 2 for expressing an exogenous gene.

4. The use according to claim 3, **characterized in that** the exogenous gene encodes an antigenic protein or polypeptide of microorganisms such as viruses or bacterium, and encodes a tumor antigen, wherein the exogenous gene is selected from the group consisting of a DNA sequence encoding human papillomavirus L1 protein (HPVL1), a gene encoding human rhinovirus 3C protease (HRV 3C, abbreviated as 3C protease), a gene encoding human enterovirus 71 (EV71) capsid protein, or a gene encoding norovirus (NoV) capsid protein.

5. The use according to claim 4, **characterized in that** a host cell used for expression is *Escherichia coli* including, but not limited to DH5α, GI698, ER2566, BL21(DE3), XA90, B834(DE3) or BLR(DE3).

6. A method for constructing an expression vector by genetic recombination, comprising the steps of:
S1. designing mutagenic primers according to an SD sequence, wherein the SD sequence is AGGAGATATA, AGGAAACAGTA, AGGAGGAATAA, and AGGAGGAATTA;
S2. performing mutagenic polymerase chain reaction (PCR) using a pGEX vector as a template to introduce an NdeI restriction site into the original vector;
S3. removing GST gene sequence by NdeI and BamHI double digestion;
S4. filling in cohesive ends with DNA polymerase I and Klenow fragment;
S5. ligating with T4 DNA ligase to obtain a closed-circular plasmid;
S6. designing primers for PCR to reintroduce the NdeI restriction site into the plasmid; and
wherein the method further comprise:
S7. expanding a clone with a correct sequence, preserving the bacterial strain, and extracting and storing the plasmid.

7. A method for expressing a human papillomavirus vaccine antigen using the plasmid expression vector according to claim 1 or 2, **characterized by**: cloning a gene encoding the antigen into the plasmid expression vector and expressing the antigen in a host cell; wherein the antigen is an L1 protein selected from the group consisting of HPV6, HPV11, HPV16, HPV18, HPV31, HPV33, HPV35, HPV39, HPV45, HPV51, HPV52, HPV56, HPV58, HPV59 and HPV68; and
wherein the L1 protein is modified by truncation of one or more amino acids at the N-terminus, one or more amino acids at the C-terminus, or one or more amino acids at both the N-terminus and the C-terminus.

8. The method according to claim 7, **characterized in that** the amino acid sequence of the L1 protein comprises SEQ ID No. 1, SEQ ID No. 2, SEQ ID No. 3, SEQ ID No. 4, SEQ ID No. 5, SEQ ID No. 6, SEQ ID No. 7, SEQ ID No. 8, SEQ ID No. 9, SEQ ID No. 10, SEQ ID No. 11, SEQ ID No. 12, SEQ ID No. 13, SEQ ID No. 14 or SEQ ID No. 15.

9. The method according to claim 7 or 8, **characterized in that** the host cell is *Escherichia coli* including, but not limited to DH5α, GI698, ER2566, BL21(DE3), XA90, B834(DE3) or BLR(DE3).

10. The method according to claim 9, **characterized by** further comprising the steps of collecting and purifying the antigen.
